(19) Europäisches Patentamt / European Patent Office / Office européen des brevets

(11) **EP 4 576 100 A1**

(12) **EUROPEAN PATENT APPLICATION**
published in accordance with Art. 153(4) EPC

(43) Date of publication:
**25.06.2025 Bulletin 2025/26**

(21) Application number: **23854737.6**

(22) Date of filing: **07.07.2023**

(51) International Patent Classification (IPC):
**G16B 15/30** (2019.01)

(52) Cooperative Patent Classification (CPC):
**G16B 15/30**

(86) International application number:
**PCT/JP2023/025264**

(87) International publication number:
**WO 2024/038700 (22.02.2024 Gazette 2024/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.08.2022  JP 2022129776**

(71) Applicant: **FUJIFILM Corporation**
**Tokyo 106-8620 (JP)**

(72) Inventors:
• **TSUMURA Kyosuke**
  **Ashigarakami-gun, Kanagawa 258-8577 (JP)**
• **NAKABAYASHI Jun**
  **Ashigarakami-gun, Kanagawa 258-8577 (JP)**

(74) Representative: **Klunker IP**
**Patentanwälte PartG mbB**
**Destouchesstraße 68**
**80796 München (DE)**

(54) **COMPOUND SEARCHING DEVICE, COMPOUND SEARCHING METHOD, AND COMPOUND SEARCHING PROGRAM**

(57)    An aspect of the present invention provides a compound searching device, a compound searching method, and a compound searching program that can efficiently search for a low-molecular-weight compound having activity and membrane permeability. **In a** compound searching device according to an aspect of the present invention, a processor specifies a side of a part of a ligand of which a proportion in a bonding force of the ligand to a target protein is equal to or higher than a designated value as a main site, and calculates a partial feature amount derived from the main site in a feature amount related to a degree of accumulation of an amino acid with respect to the ligand. **In** addition, the processor calculates a similarity between a feature amount for a search target compound and the partial feature amount. The processor outputs the similarity of the search target compound and information indicating a molecular weight and/or activity of the search target compound in association with each other to a recording device and/or a display device.

FIG. 4

START

ACQUIRE STRUCTURAL INFORMATION OF SEARCH TARGET COMPOUND ~S100

ACQUIRE CONFORMATIONAL INFORMATION OF LIGAND ~S110

CALCULATE OVERALL FEATURE AMOUNT OF LIGAND ~S120

SPECIFY SITE OF PART OF LIGAND AS MAIN SITE ~S130

CALCULATE PARTIAL FEATURE AMOUNT DERIVED FROM MAIN SITE ~S140

CALCULATE OVERALL FEATURE AMOUNT OF SEARCH TARGET COMPOUND ~S150

CALCULATE SIMILARITY BETWEEN PARTIAL FEATURE AMOUNT OF LIGAND AND OVERALL FEATURE AMOUNT OF SEARCH TARGET COMPOUND ~S160

OUTPUT SIMILARITY AND INFORMATION INDICATING MOLECULAR WEIGHT AND/OR ACTIVITY IN ASSOCIATION WITH EACH OTHER ~S170

EXTRACT COMPOUND HAVING SIMILARITY EQUAL TO OR HIGHER THAN THRESHOLD VALUE FROM SEARCH TARGET COMPOUNDS ~S180

END

**Description**

**BACKGROUND OF THE INVENTION**

1. Field of the Invention

The present invention relates to a compound searching device, a compound searching method, and a compound searching program, and particularly relates to a technique for searching for or designing another ligand having a reduced molecular weight from a ligand such as a non-peptide natural product, a peptide, or a protein.

2. Description of the Related Art

A protein-protein interaction (PPI) is a frontier of drug discovery. In fact, there are about 30,000 important and unexamined PPIs. Antibodies and the like (which may be antibodies or medium molecular weight peptides; the same applies hereinafter) have been studied as molecules that control PPI, but membrane permeability is low due to the molecular weight even though they have activity, and the development of the present frontier is limited in a case of targeting intracellular targets or targeting brain drugs or oral drugs even in a case of targeting extracellular targets.
As described above, imparting membrane permeability to an antibody or the like is essential for the development of the present frontier. As one of the measures for imparting membrane permeability, it has been proposed to reduce a molecular weight of an antibody or the like (for example, see "Constrained Peptides in Drug Discovery and Development", Douglas R. Cary et al., Journal of Synthetic Organic Chemistry, Japan, Volume 75 (2017) Issue 11, [searched on July 29, 2022], Internet (https://www.jstage.jst.go.jp/article/yukigoseikyokaishi/75/11/75_1171/_pdf/-char/en)). The reduction of a molecular weight is a technique for searching for or designing a compound that is generated as a low-molecular-weight compound having a molecular weight of about 500, in which an unnecessary site for the activity of an antibody or the like is discarded and membrane permeability can be expected.
The reduction of a molecular weight of an antibody or the like or, more generally, skeleton conversion of a compound into another compound has been studied previously by several methods. For example, in a peptidomimetic method (see WO2009/148192A, WO2010/044485A, and WO2010/128685A), a non-peptide structure that reproduces a secondary structure (such as an α-helix or a β-sheet) of a protein is created in advance, and a side chain of the antibody or the like that is important for interaction with a target is introduced into the non-peptide structure under a condition that the side chain is spatially located at the same position as the non-peptide structure, thereby producing a low-molecular-weight compound.
In addition, in a pharmacophore method (see "Translating peptides into small molecules", Gerd Hummel et al., Molecular BioSystems, 2006, 2, 499-508, [searched on July 29, 2022], Internet (https://pubs.rsc.org/en/content/articlehtml/2006/mb/b611791k)), a low-molecular-weight compound was created by leaving only partial structures of the antibody or the like that are important for interaction with a target, abstracting the partial structures according to the type of interaction (hydrogen bond donor, acceptor, hydrophobic interaction site, and the like), and searching for or designing a compound under a condition that the compound has the same type of interaction at the same spatial position as the abstracted partial structures.
In addition, in a structure-based drug design (SBDD) method (see WO2006/099178A), bonding energy correlated with activity can be calculated by molecular dynamics (MD) simulation based on fundamental principles for a three-dimensional structure of a target obtained by analyzing a complex structure of the antibody or the like and the target, and a low-molecular-weight compound was created by searching for or designing a compound under a condition that the bonding energy is high.
In addition, in an amino acid mapping (AAM) method (see JP6826672B), an AAM descriptor can be calculated by calculating an interaction with an amino acid based on fundamental principles for the compound, and another compound was created by searching for or designing a compound under a condition that a similarity with the AAM descriptor, which is correlated with activity, is high.

**SUMMARY OF THE INVENTION**

However, in the peptidomimetic method described in WO2009/148192A, WO2010/044485A, and WO2010/128685A, a portion of the antibody or the like including a residue that is important for interaction with a target is required to be a secondary structure of a protein, and the structure of the antibody or the like that can be applied is limited. In the pharmacophore method described in "Translating peptides into small molecules", Gerd Hummel et al., Molecular BioSystems, 2006, 2, 499-508, [searched on July 29, 2022], Internet (https://pubs.rsc.org/en/content/articlehtml/2006/mb/b611791k), there is no restriction on the structure of the antibody or the like in the peptidomimetic method, but the classification of the type of interaction, which is a feature of this technique, needs to be set in advance by a person, and there is uncertainty in the classification of the type of interaction.

In addition, in the SBDD method described in WO2006/099178A, there is no uncertainty in the classification of the type of interaction in the pharmacophore method, but the calculation of the bonding energy in the MD simulation takes time because information on a target having a large molecular weight is used, and a chemical space of a compound as a target for search and design is limited. In addition, in the AAM method described in JP6826672B, there is no restriction on the chemical space due to an increase in calculation time caused by handling a target in the SBDD method, but there is a problem that a molecular weight hardly changes upon the skeleton conversion.

As described above, a technique for systematically executing reduction of a molecular weight of an antibody or the like does not currently exist, and there is a demand for development of such a method.

The present invention has been made in view of such circumstances, and an object of the present invention is to provide a compound searching device, a compound searching method, and a compound searching program that can efficiently search for a low-molecular-weight compound having activity and membrane permeability.

In order to achieve the above-described object, according to a first aspect of the present invention, there is provided a compound searching device comprising a processor, in which the processor is configured to: acquire information indicating a three-dimensional structure of a search target compound; acquire information indicating a conformation of a ligand in a complex structure of the ligand and a target protein; calculate a first feature amount that is a feature amount obtained by quantifying, in a three-dimensional space, a degree of accumulation of one or more amino acids as a probe for the ligand in a periphery of the ligand with respect to the conformation of the ligand; specify, as a main site, a site of a part of the ligand, of which a proportion in a bonding force of the ligand to the target protein is equal to or higher than a first threshold value; calculate a partial feature amount that is a part derived from the main site, in the first feature amount; calculate a second feature amount that is a feature amount obtained by quantifying, in a three-dimensional space, a degree of accumulation of one or more amino acids as a probe for the search target compound in a periphery of the search target compound; calculate a similarity between the second feature amount for the search target compound and the partial feature amount of the ligand; and output the similarity for the search target compound and information indicating a molecular weight and/or activity of the search target compound in association with each other to a recording device and/or a display device.

According to the first aspect, the "partial feature amount" is a feature amount derived from the main site in a feature amount derived from the entire ligand, and is likely to have a smaller value than the first feature amount. Then, in the first aspect, the similarity between the "partial feature amount" and the second feature amount derived from the entire search target compound is calculated, and a compound having a high similarity (equal to or higher than a second threshold value) is calculated. Since the compound having a high similarity has a feature amount (second feature amount) having a value smaller than (or having a high possibility of being smaller than) the first feature amount, the compound having a high similarity is likely to have a smaller molecular weight than the original ligand. In the first aspect, the similarity of the search target compound and the information indicating the molecular weight and/or the activity of the search target compound are output in association with each other to the recording device and/or the display device.

As described above, since the reduction in molecular weight is one of the measures for imparting activity and membrane permeability, according to the first aspect, it is possible to efficiently search for a low-molecular-weight compound having activity and membrane permeability. In the first aspect, the processor may extract a compound satisfying a desired condition from the search target compound with reference to the output information (the similarity of the search target compound and the information indicating the molecular weight and/or the activity of the search target compound).

In the first aspect and each of the following aspects, the first feature amount for the ligand may be referred to as an "overall feature amount" of the ligand, and the second feature amount for the search target compound may be referred to as an "overall feature amount" of the search target compound.

In the first aspect and each of the following aspects, the processor may use a compound that is acquired in advance and recorded on a recording medium as the "search target compound", or may search for a compound recorded in a library each time the processing is performed. In addition, the processor may specifically designate the search target compound (according to an operation of a user or without depending on the operation of the user) in the processing. Such a recording medium and library may be included in the compound searching device according to the first aspect, or may be recording media and libraries outside the device.

In addition, in the first aspect and each of the following aspects, the "information indicating a three-dimensional structure" may be the three-dimensional structure itself or may be information from which the three-dimensional structure can be indirectly obtained. For example, in a case where a structural formula is acquired as the "information indicating a three-dimensional structure", the three-dimensional structure of the compound can be obtained from the structural formula by various methods.

According to a second aspect, in the compound searching device according to the first aspect, the processor is configured to, in specifying the main site, acquire any one of a result of bonding energy calculation using a complex structure of the ligand and the target protein, information indicating a structural activity correlation between the ligand and a ligand analogous substance, or the first feature amount for a local stable conformation of the ligand from a non-transitory and tangible recording medium, as the information indicating the bonding force, and specify the main site based on the acquired information. The second aspect defines one aspect of a technique for specifying the main site.

In the second aspect, examples of the "non-transitory and tangible recording medium" include various magneto-optical recording devices and semiconductor memories. The "non-transitory and tangible recording medium" does not include a non-tangible recording medium such as a carrier wave signal itself and a propagation signal itself.

According to a third aspect, in the compound searching device according to the first or second aspect, the processor is configured to, in specifying the main site, select one or more amino acid residues in which a ratio of a total value of a breakdown of a bonding force due to each amino acid residue of the ligand to the bonding force of the ligand is equal to or higher than the first threshold value, and specify a site of the one or more amino acid residues in the ligand as the main site. The third aspect specifically defines one aspect of a technique for specifying the main site.

According to a fourth aspect, in the compound searching device according to the third aspect, the processor is configured to, in specifying the main site, select the one or more amino acid residues in consideration of a distance between the amino acid residues. The fourth aspect more specifically defines the technique for specifying the main site in the third aspect.

According to a fifth aspect, in the compound searching device according to the first or second aspect, the processor is configured to, in specifying the main site, specify a site of the ligand present in a designated region as the main site. In the fifth aspect, the processor may designate the region according to the operation of the user, or may designate the region without depending on the operation of the user.

According to a sixth aspect, in the compound searching device according to the fifth aspect, the processor is configured to, in specifying the main site, designate the region based on a distribution of the first feature amount in a periphery of the ligand. In the sixth aspect, for example, the processor can designate a region including a portion in which the degree of accumulation is maximized.

According to a seventh aspect, in the compound searching device according to the fifth aspect, the processor is configured to, in specifying the main site, determine at least one of the number, position, size, or shape of the region according to an operation of a user via an input device. According to the seventh aspect, the user can designate at least one of the number, position, size, or shape of the region.

According to an eighth aspect, in the compound searching device according to the fifth aspect, the processor is configured to, in specifying the main site, display, on the display device, information indicating a three-dimensional structure of the ligand, a distribution of the first feature amount in a periphery of the ligand, and the number, position, size, and shape of the region in a superimposed manner. According to the eighth aspect, the user can easily ascertain a relationship between the degree of accumulation and the region by this display.

According to a ninth aspect, in the compound searching device according to the first or second aspect, the processor is configured to display information indicating a ratio of the partial feature amount to the first feature amount on the display device. According to the ninth aspect, the user can easily ascertain the ratio of the partial feature amount to the first feature amount.

According to a tenth aspect, in the compound searching device according to the third aspect, the processor is configured to, in specifying the main site, display, on the display device, the site of the one or more amino acid residues specified as the main site in a three-dimensional structure of the ligand in an identifiable manner. According to the tenth aspect, the user can easily ascertain the site of the amino acid residue specified as the main site.

According to an eleventh aspect, in the compound searching device according to the first or second aspect, the processor is configured to, in specifying the main site, calculate a breakdown of bonding energy between the ligand and the target protein for each amino acid residue constituting the ligand, and display the calculated breakdown on the display device for each amino acid residue. According to the eleventh aspect, the user can easily ascertain the bonding energy by each residue.

According to a twelfth aspect, in the compound searching device according to the first or second aspect, the processor is configured to: calculate a plurality of partial feature amounts by translating and/or rotating the partial feature amount of the ligand in the calculation of the partial feature amount; and calculate the similarity based on a cosine similarity or a Euclidean distance between the plurality of partial feature amounts and the second feature amount for the search target compound in the calculation of the similarity. The twelfth aspect specifically defines one aspect of a technique for calculating the similarity.

According to a thirteenth aspect, in the compound searching device according to the first or second aspect, the processor is configured to, in the outputting, extract a compound of which the similarity is equal to or higher than a second threshold value from the search target compounds, and output information indicating the extracted compound to the recording device and/or the display device. In the thirteenth aspect, the processor may extract a predetermined number of compounds in descending order of the similarity, or may extract all the compounds having a similarity equal to or higher than the second threshold value. The processor may determine the number of compounds to be extracted according to the operation of the user.

In order to achieve the above-described object, according to a fourteenth aspect of the present invention, there is provided a compound searching method executed by a compound searching device including a processor, the method comprising: via the processor, acquiring information indicating a three-dimensional structure of a search target compound; acquiring information indicating a conformation of a ligand in a complex structure of the ligand and a target protein; calculating a first feature amount that is a feature amount obtained by quantifying, in a three-dimensional space, a degree of accumulation of one or more amino acids as a probe for the ligand in a periphery of the ligand with respect to the conformation of the ligand; specifying, as a main site, a site of a part of the ligand, of which a proportion in a bonding force of the ligand to the target protein is equal to or higher than a first threshold value; calculating a partial feature amount that is a part derived from the main site, in the first feature amount; calculating a second feature amount that is a feature amount obtained by quantifying, in a three-dimensional space, a degree of accumulation of one or more amino acids as a probe for the search target compound in a periphery of the search target compound; calculating a similarity between the second feature amount for the search target compound and the partial feature amount of the ligand; and outputting the similarity for the search target compound and information indicating a molecular weight and/or activity of the search target compound in association with each other to a recording device and/or a display device.

According to the fourteenth aspect, similarly to the first aspect, it is possible to efficiently search for a low-molecular-weight compound having activity and membrane permeability. The compound searching method according to the fourteenth aspect may have the same configurations as those of the second to thirteenth aspects.

In order to achieve the above-described object, according to a fifteenth aspect of the present invention, there is provided a compound searching program for causing a compound searching device including a processor to execute a compound searching method, the compound searching method comprising: via the processor, acquiring information indicating a three-dimensional structure of a search target compound; acquiring information indicating a conformation of a ligand in a complex structure of the ligand and a target protein; calculating a first feature amount that is a feature amount obtained by quantifying, in a three-dimensional space, a degree of accumulation of one or more amino acids as a probe for the ligand in a periphery of the ligand with respect to the conformation of the ligand; specifying, as a main site, a site of a part of the ligand, of which a proportion in a bonding force of the ligand to the target protein is equal to or higher than a first threshold value; calculating a partial feature amount that is a part derived from the main site, in the first feature amount; calculating a second feature amount that is a feature amount obtained by quantifying, in a three-dimensional space, a degree of accumulation of one or more amino acids as a probe for the search target compound in a periphery of the search target compound; calculating a similarity between the second feature amount for the search target compound and the partial feature amount of the ligand; and outputting the similarity for the search target compound and information indicating a molecular weight and/or activity of the search target compound in association with each other to a recording device and/or a display device.

According to the fifteenth aspect, similarly to the first and fourteenth aspects, it is possible to efficiently search for a low-molecular-weight compound having activity and membrane permeability. The compound searching program according to the fifteenth aspect may have the same configuration as those of the second to thirteenth aspects.

In addition, a non-transitory and tangible recording medium (for example, various magneto-optical recording devices and semiconductor memories) on which a computer-readable code of the program of these aspects is recorded can also be mentioned as an aspect of the present invention. The "non-transitory and tangible recording medium" described above does not include a non-tangible recording medium such as the carrier wave signal itself and the propagation signal itself.

As described above, according to the compound searching device, the compound searching method, and the compound searching program of the present invention, it is possible to efficiently search for a low-molecular-weight compound having activity and membrane permeability.

**BRIEF DESCRIPTION OF THE DRAWINGS**

Fig. 1 is a diagram showing a configuration of a compound searching device.
Fig. 2 is a diagram showing a functional configuration of a processing unit.
Fig. 3 is a diagram showing information stored in a storage unit.
Fig. 4 is a flowchart showing processing of a compound searching method.
Fig. 5 is a flowchart showing a method of calculating a three-dimensional AAM descriptor.
Figs. 6A and 6B are diagrams showing a state in which a structural formula is three-dimensionalized.
Figs. 7A and 7B are diagrams showing an example of the three-dimensional AAM descriptor.
Fig. 8 is another diagram showing an example of the three-dimensional AAM descriptor.
Figs. 9A, 9B, and 9C are diagrams showing a state in which a main site is specified.
Fig. 10 is a diagram showing an example of display of a contribution rate of an interaction due to a selected residue.

Figs. 11A, 11B, and 11C are diagrams showing a state in which a partial feature amount is calculated.
Figs. 12A, 12B, and 12C are diagrams showing a state in which an overall feature amount of a search target compound is calculated.
Figs. 13A and 13B are diagrams showing a state in which a similarity between feature amounts is calculated.
Fig. 14 is a diagram showing names and molecular weights of the search target compounds.
Fig. 15 is a diagram showing a relationship between a similarity of a feature amount and an activity.
Figs. 16A and 16B are diagrams showing a similarity between an overall feature amount of a ligand and a partial feature amount and an overall feature amount of the search target compound.

## DESCRIPTION OF THE PREFERRED EMBODIMENTS

Embodiments of a compound searching device, a compound searching method, and a compound searching program according to the present invention will be described in detail. In the description, the accompanying drawings will be referred to as necessary.

[First Embodiment]

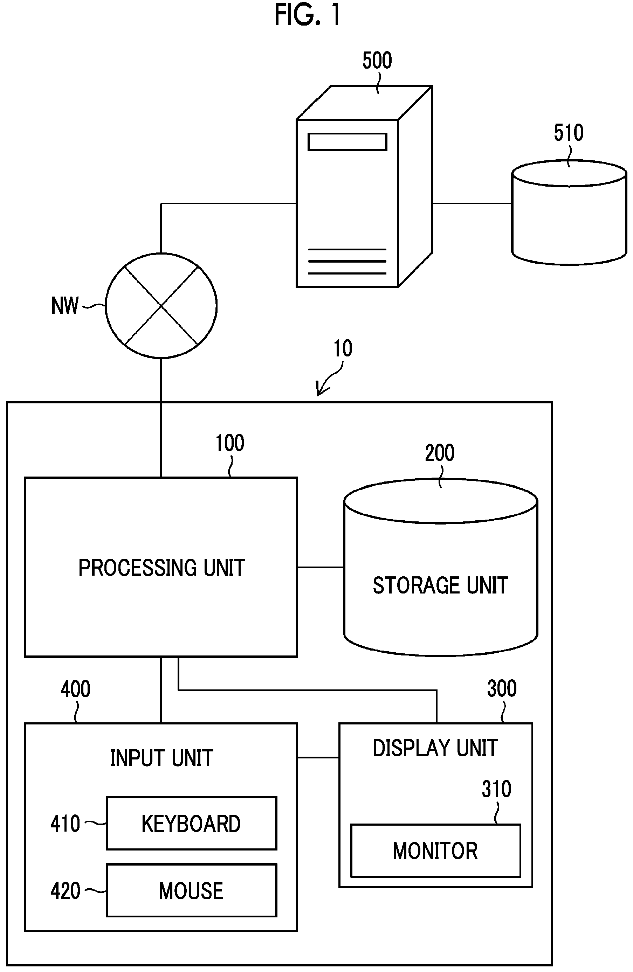

Fig. 1 is a block diagram showing a configuration of a compound searching device 10 (compound searching device) according to a first embodiment. The compound searching device 10 is a device that performs calculation of feature amounts (a first feature amount, a partial feature amount, and a second feature amount) for a ligand and a compound (target structures) and performs extraction (screening) of a compound based on the feature amounts, and can be realized by using a computer. As shown in Fig. 1, the compound searching device 10 comprises a processing unit 100, a storage unit 200, a display unit 300, and an operation unit 400 (input device), which are connected to each other to transmit and receive necessary information. These components can be installed by employing various forms, and the components may be installed in one place (in one housing, one room, or the like) or may be installed in places separated from each other and connected via a network. In addition, the compound searching device 10 is connected to an external server 500 and an external database 510 such as a protein data bank (PDB) via a network NW such as the Internet, and can acquire information (for example, a result of X-ray co-crystal structure analysis or nuclear magnetic resonance (NMR) structure analysis) such as a structural formula of a compound and a crystal structure of a protein, as necessary.

[Configuration of Processing Unit]

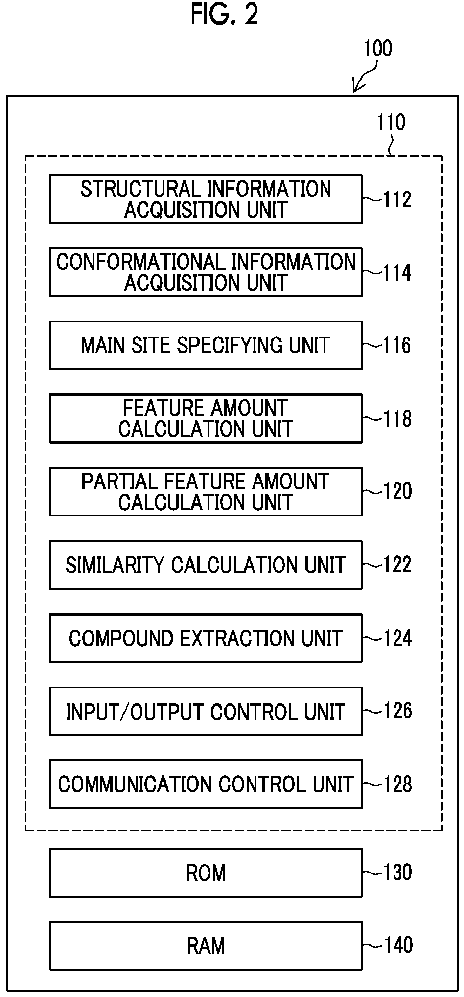

Fig. 2 is a diagram showing a configuration of the processing unit 100. The processing unit 100 comprises a processor 110 (processor), a read only memory (ROM) 130, and a random access memory (RAM) 140. The processor 110 has functions as a structural information acquisition unit 112, a conformational information acquisition unit 114, a main site specifying unit 116, a feature amount calculation unit 118, a partial feature amount calculation unit 120, a similarity calculation unit 122, a compound extraction unit 124, an input/output control unit 126, and a communication control unit 128.

[Overview of Functions of Processing Unit]

The structural information acquisition unit 112 acquires information indicating a three-dimensional structure of a search target compound. The conformational information acquisition unit 114 acquires information indicating a conformation of a ligand in a complex structure of the ligand and a target protein. The main site specifying unit 116 specifies a site of a part of the ligand as a main site. The feature amount calculation unit 118 calculates a feature amount (first feature amount) for the ligand and the search target compound. The partial feature amount calculation unit 120 calculates a partial feature amount of the ligand. The similarity calculation unit 122 calculates a similarity between an overall feature amount (second feature amount) for the search target compound and the partial feature amount of the ligand. The compound extraction unit 124 extracts a compound having a similarity equal to or higher than a second threshold value from the search target compound.

The input/output control unit 126 controls input and output of information to and from the storage unit 200, reception of a user operation via the operation unit 400, and output of information to the display unit 300. The communication control unit 128 controls communication with devices such as the external server 500 and/or the external database 510 via the network NW in cooperation with each of the above-described units. For example, the input/output control unit 126 and the communication control unit 128 may execute various calculations (by methods such as MO, MD, MM, and AlphaFold2) regarding the structure and/or the properties of the compound on a server or a computer, such as the external server 500, and acquire results thereof (or only results that have already been executed). In addition, the compound searching device 10 itself may have a function of performing such calculation.

The function of each unit of the processing unit 100 (processor 110) described above can be realized by using various processors. The various processors include, for example, a central processing unit (CPU) which is a general-purpose processor that realizes various functions by executing software (programs). The various processors described above also include a programmable logic device (PLD), which is a processor whose circuit configuration can be changed after manufacturing, such as a field programmable gate array (FPGA). Further, the various processors described above also include a dedicated electric circuit, which is a processor having a circuit configuration specifically designed to execute specific processing such as an application specific integrated circuit (ASIC).

The function of each unit may be realized by one processor or a combination of a plurality of processors. A plurality of functions may be realized by one processor. As an example of configuring the plurality of functions by one processor, firstly, there is a form in which one processor is configured by a combination of one or more central processing units (CPUs) and software and the processor realizes the plurality of functions, as represented by a computer such as a client or a server. Secondly, there is a form in which a processor that realizes functions of the entire system by one integrated circuit (IC) chip is used, as represented by a system on chip (SoC). As described above, various functions are configured using one or more of the above-described various processors as a hardware structure. Further, as the hardware structure of the various processors, more specifically, an electric circuit (circuitry) in which circuit elements such as semiconductor elements are combined is used.

In a case where the above-described processor or electric circuit executes software (program), a processor (computer)-readable code of the software to be executed is stored in a non-transitory and tangible recording medium such as the ROM 130, and the processor refers to the software. The software stored in the non-transitory and tangible recording medium includes a program (compound searching program) for executing the compound searching method according to the embodiment of the present invention. The code may be recorded on a non-transitory and tangible recording medium, such as various magneto-optical recording devices and semiconductor memories, instead of the ROM 130. In a case of executing processing using software, for example, the RAM 140 is used as a transitory storage area, and a program and/or data stored in a non-volatile memory (non-transitory and tangible recording medium), such as an electrically erasable and programmable read only memory (EEPROM) and a flash memory (not shown), may be referred to.

The "non-transitory and tangible recording medium" described above does not include a non-tangible recording medium such as a carrier wave signal itself and a propagation signal itself.

Details of the processing using each of these units of the processing unit 100 will be described later.

[Configuration of Storage Unit]

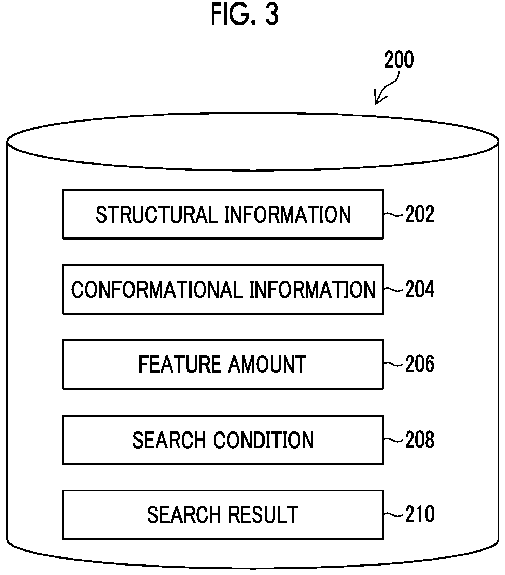

The storage unit 200 is configured by a non-transitory and tangible recording medium, such as various magneto-optical recording media and semiconductor memories, and an input/output control unit thereof. In the storage unit 200 according to the present embodiment, for example, as shown in Fig. 3, structural information 202 (structural information of the search target compound), conformational information 204 (conformational information of the ligand), a feature amount 206 (an overall feature amount (first feature amount) and a partial feature amount of the ligand, and an overall feature amount (second feature amount) of the search target compound), a search condition 208, and a search result 210 are stored in association with each other.

[Configuration of Display Unit and Operation Unit]

The display unit 300 includes a monitor 310 (display device), and can display input information, information stored in the storage unit 200, a result of processing by the processing unit 100, and the like. The operation unit 400 (input device) includes a keyboard 410 and a mouse 420 as an input device and/or a pointing device, and a user can perform an operation necessary for executing the compound searching method and the compound searching program according to the embodiment of the present invention via these devices and a screen of the monitor 310.

[Processing of Compound Searching Method and Compound Searching Program]

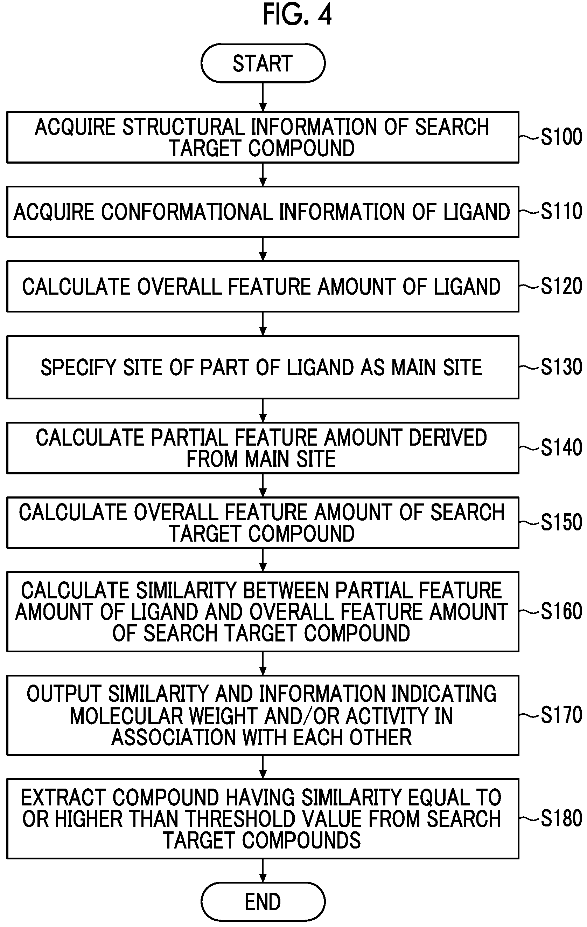

Hereinafter, the compound searching method and the compound searching program in the compound searching device 10 having the above-described configuration will be described with reference to the flowchart of Fig. 4 and the like.

[Acquisition of Structural Information]

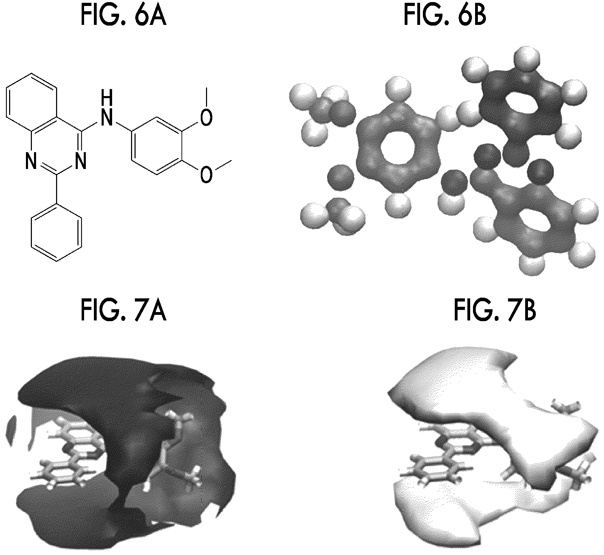

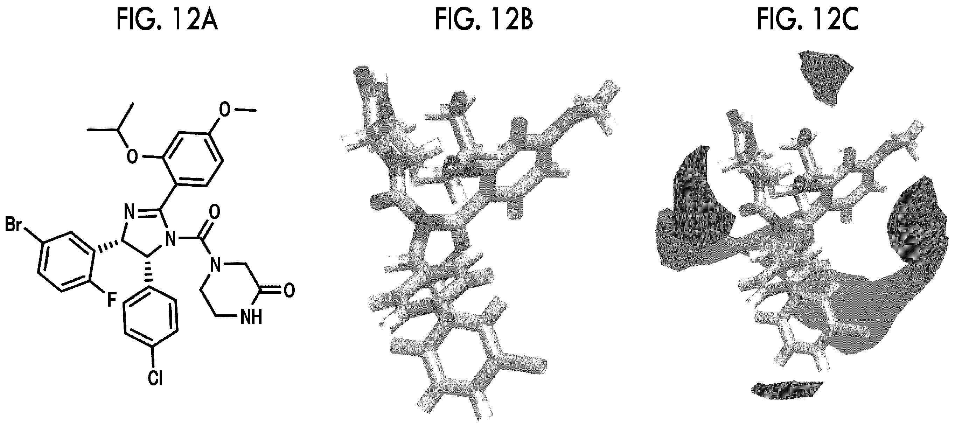

The structural information acquisition unit 112 (processor) acquires information indicating the three-dimensional structure of the search target compound (step S100: structural information acquisition processing, structural information acquisition step). The structural information acquisition unit 112 may acquire the information indicating the three-dimensional structure from the structural information 202 (storage unit 200), the external server 500, or the external database 510, or may acquire the information indicating the three-dimensional structure through a user operation via the operation unit 400. In addition, the "information indicating the three-dimensional structure" may be the three-dimensional structure itself or may be information from which the three-dimensional structure can be indirectly obtained. The structural information acquisition unit 112 can acquire, for example, a structural formula as the "information indicating the three-dimensional structure" and obtain the three-dimensional structure of the compound from the structural formula (see examples in Figs. 6A and 6B and Figs. 12A, 12B, and 12C described later).

[Acquisition of Conformational Information]

The conformational information acquisition unit 114 (processor) acquires information (conformational information) indicating the conformation (bonding conformation) of the ligand in the complex structure of the ligand and the target protein (step S114: conformational information acquisition processing, conformational information acquisition step). Examples of the conformational information include the following information.

(1) Results of X-ray co-crystal structure analysis or nuclear magnetic resonance (NMR) structure analysis of a ligand and a target protein
(2) Results of docking simulation of a ligand with respect to a structure of a target protein alone obtained by X-ray co-crystal structure analysis or NMR structure analysis of another ligand and a target protein
(3) Results of docking simulation of a ligand with respect to a structure of a target protein alone obtained by X-ray crystal structure analysis, NMR structure analysis, or folding calculation (using MD, MM, AlphaFold2, or the like) of the target protein alone (MD: Molecular Dynamics, MM: Molecular Mechanics, and AlphaFold2 is a protein structure calculation tool available on GitHub).
(4) Results of X-ray crystal structure analysis, NMR structure analysis, and search calculation for a local stable conformation (using MD, MM, AlphaFold2, or the like) of a ligand alone

[Calculation of Overall Feature Amount of Ligand (First Feature Amount)]

The feature amount calculation unit 118 (processor) calculates a first feature amount, which is a feature amount obtained by quantifying, in a three-dimensional space, a degree of accumulation of one or more amino acids as a probe for the ligand in a periphery of the ligand with respect to the conformation of the ligand acquired in step S110 (step S120: first feature amount calculation processing, first feature amount calculation step). The first feature amount is a feature amount derived from the entire ligand, and hereinafter, may be referred to as an "overall feature amount" of the ligand.

[Method of Calculating First Feature Amount (Three-dimensional AAM Descriptor)]

The first feature amount is a "three-dimensional AAM descriptor" (AAM: Amino Acid Mapping) described in JP6826672B, and the feature amount calculation unit 118 (processor) can calculate the three-dimensional AAM descriptor by the method described in JP6826672B. Hereinafter, a method of calculating the three-dimensional AAM descriptor will be specifically described. In the present invention, the entire content of JP6826672B is incorporated by reference, including the matters described below.

(Method 1)

A feature amount calculation method of Method 1 is a feature amount calculation method executed by a feature amount calculation device including a processor, in which the processor executes a target structure designation step of designating a target structure which is composed of a plurality of unit structures having chemical properties, a three-dimensional structure generation step of generating a three-dimensional structure with the plurality of unit structures for the target structure, and a feature amount calculation step of calculating a feature amount obtained by quantifying, in a three-dimensional space, a degree of accumulation of one or more kinds of probes in a periphery of the three-dimensional structure, and the probe is a structure in which a plurality of points having a real electric charge and generating a van der Waals force are disposed to be separated from each other.

(Method 2)

According to Method 2, in Method 1 above, the processor designates a compound as the target structure in the target structure designation step, generates a three-dimensional structure of the compound with a plurality of atoms in the three-dimensional structure generation step, and calculates a three-dimensional AAM descriptor which is a feature amount obtained by quantifying, in a three-dimensional space, a degree of accumulation of amino acids as probes in a periphery of the three-dimensional structure of the compound generated in the three-dimensional structure generation step in the feature amount calculation step.

In Method 2 above, the feature amount calculation unit 118 (processor) can calculate a three-dimensional AAM descriptor of a ligand by designating the ligand as the "target structure", and this "three-dimensional AAM descriptor" corresponds to the "first feature amount" in the present invention. Hereinafter, a method of calculating the three-dimensional AAM descriptor will be specifically described. In the following, the feature amount calculated by Method 2 may be referred to as a "three-dimensional AAM descriptor" or simply an "AAM descriptor".

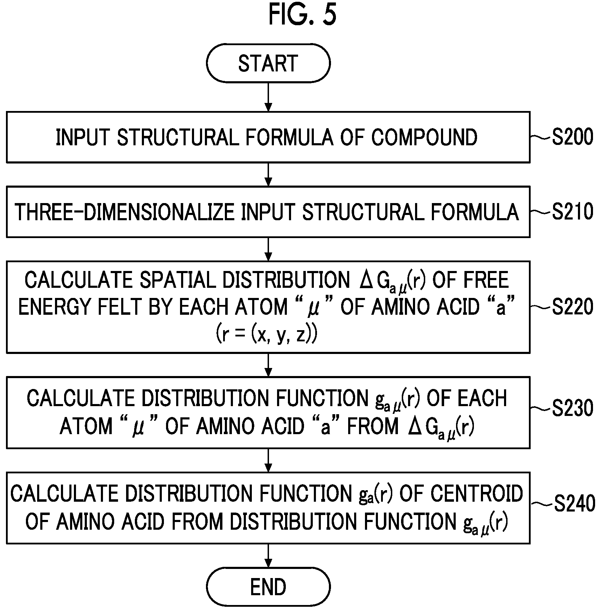

The compound searching device 10 can calculate the three-dimensional AAM descriptor in response to an instruction from the user via the operation unit 400. Fig. 5 is a flowchart showing a procedure for calculating a three-dimensional AAM descriptor for a compound (target structure). The ligand is a compound whose bonding to the target protein has been confirmed, and the three-dimensional AAM descriptor can be calculated according to the procedure of Fig. 5. In step S200, the structural information acquisition unit 112 inputs the structural formula (an example of information indicating the three-dimensional structure) of the compound according to the operation of the user. As a result, the compound represented by the input chemical formula is designated as the target structure (target structure designation processing, target structure designation step).

The feature amount calculation unit 118 three-dimensionalizes the input structural formula to generate a three-dimensional structure of a compound with a plurality of atoms (a plurality of unit structures having chemical properties) (step S210: three-dimensional structure generation processing, three-dimensional structure generation step). Various techniques are known for three-dimensionalization of a structural formula, and the technique used in step S210 is not particularly limited. Figs. 6A and 6B are an example of three-dimensionalization of a structural formula, in which Fig. 6A shows the input structural formula and Fig. 6B shows the three-dimensionalized structural formula (it should be noted that Figs. 6A and 6B show states viewed from opposite sides).

The feature amount calculation unit 118 calculates a spatial distribution $\Delta G_{a\mu}(r)$ of free energy felt by each atom "$\mu$" of an amino acid "a" (a represents a number representing the kind of amino acid; 1 to 20) (step S220: feature amount calculation processing, feature amount calculation step). As a method of calculating $\Delta G_{a\mu}(r)$, molecular dynamics (MD) can be employed, but the present invention is not limited thereto. The amino acid for calculating the feature amount may be a predetermined kind of amino acid or may be determined according to the instruction of the user via the operation unit 400 (one or more kinds of amino acids may be used, and a plurality of kinds of amino acids may also be used). In addition, the amino acid for calculating the feature amount may be selected in consideration of the classification. In addition, there are various classifications of amino acids, and for example, a classification of "hydrophilic, hydrophobic, and special" may be used, or other classifications such as "polar, non-polar, negatively charged, and positively charged" or "basic, acidic, and neutral" may be used.

The feature amount calculation unit 118 calculates a distribution function $g_{a\mu}(r)$ of each atom "$\mu$" of the amino acid "a" from $\Delta G_{a\mu}(r)$ (step S230: feature amount calculation processing, feature amount calculation step). $g_{a\mu}(r)$ is represented by Equation (1) in a case where T is set as room temperature and $K_B$ is set as a Boltzmann constant.

$$ g_{a\mu}(\mathbf{r}) = \exp\left(-\Delta G_{a\mu}(\mathbf{r}) / K_B T\right) \qquad \cdots (1) $$

The feature amount calculation unit 118 calculates a distribution function $g_a(r)$ of a centroid of the amino acid from the distribution function $g_{a\mu}(r)$ (step S240: feature amount calculation processing, feature amount calculation step). For the calculation, $g_{a\mu}(r)$ is geometrically averaged for each atom "$\mu$". This distribution function $g_a(r)$ is a three-dimensional AAM descriptor (first feature amount) obtained by quantifying, in a three-dimensional space, the degree of accumulation of one or more kinds of amino acids "a" in the periphery of the three-dimensional structure of the compound. The feature amount calculation unit 118 can store the calculated three-dimensional AAM descriptor in the storage unit 200 in association with the structural information and/or the structural information (information indicating the three-dimensional structure) of the compound (see Fig. 3). In addition, "r" in $\Delta G_{a\mu}(r)$, $g_{a\mu}(r)$, and $g_a(r)$ is a three-dimensional position vector (r = (x, y, z)).

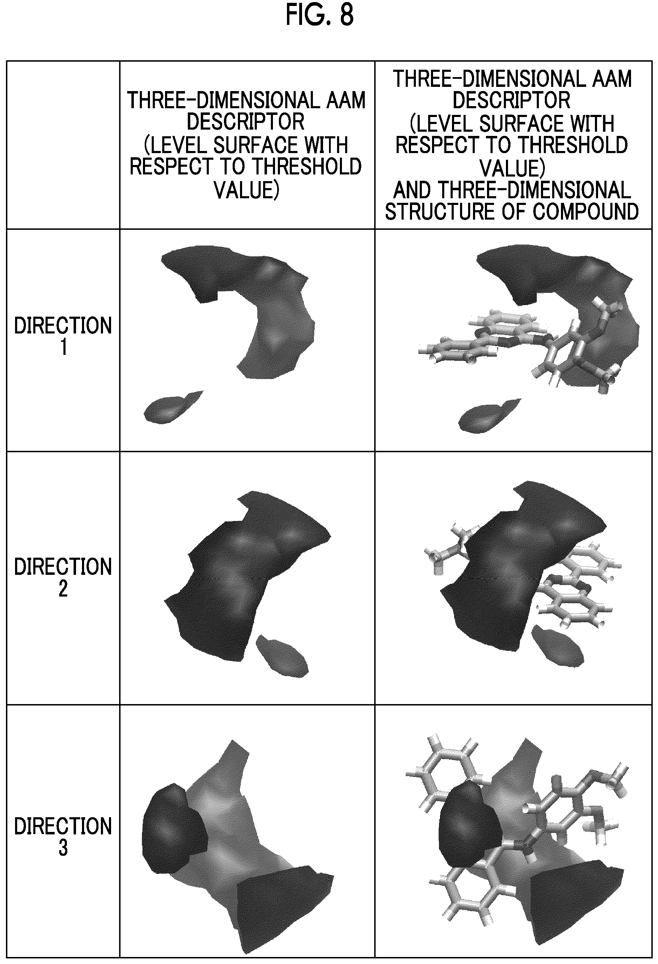

Figs. 7A and 7B are an example of a three-dimensional AAM descriptor of the compound shown in Figs. 6A and 6B. Fig. 7A shows a three-dimensional AAM descriptor for alanine, and Fig. 7B shows a three-dimensional AAM descriptor for valine. In Figs. 7A and 7B, a dark region is a region where the degree of accumulation of amino acids (existence probability) is high. Fig. 8 is a table showing another example of the three-dimensional AAM descriptor for the compound shown in Figs. 7A and 7B, and shows the three-dimensional AAM descriptors in a direction 1, a direction 2, and a direction 3 (first, second, and third rows of the table, respectively) which are different from one another. A left column of the table shows the three-dimensional AAM descriptor (level surface with respect to a threshold value), and a right column of the table shows the three-dimensional AAM descriptor (level surface with respect to the threshold value) and the three-dimensional structure of the compound.

[Specification of Main Site]

The main site specifying unit 116 (processor) specifies a site of a part of the ligand, of which a proportion in a bonding force of the ligand to the target protein is equal to or higher than a first threshold value, as a main site (step S120 in Fig. 4: main site specifying processing, main site specifying step).

The main site specifying unit 116 can specify the main site by, for example, any one of the following methods (1) to (3). The main site specifying unit 116 may specify the main site without using the "three-dimensional AAM descriptor" as in the method (1) and the method (2), or may specify the main site using the "three-dimensional AAM descriptor" as in the method (3).

(1) Using results of bonding energy calculation using a complex structure of a ligand and a target protein (using FMO, MO, MD, MM, or the like) (FMO method: Fragment Molecular Orbital method, MO method: Molecular Orbital method).
(2) Using a calculation result of a structural activity correlation between a ligand and a ligand analogous substance.
(3) Specifying a main site based on the three-dimensional AAM descriptor for a local stable conformation of the ligand alone (for example, detecting a point at which an intensity of the three-dimensional AAM descriptor is maximum and specifying a site of the ligand in the vicinity of the point as the main site)

The method of calculating the "three-dimensional AAM descriptor" (feature amount calculation method) in the method (3) is as described above. In addition, a specific example of the main site (aspect in which the bonding force due to an amino acid residue is taken into consideration in the method (1)) will be described in detail in the section of "Examples".

The main site specifying unit 116 may determine which technique is used to specify the main site through the operation of the user via the operation unit 400, or may determine without depending on the operation of the user. In addition, the main site specifying unit 116 may determine at least one of the number, position, size, or shape of regions for specifying the main site according to the operation of the user via the operation unit 400 (input device). In this case, the main site specifying unit 116 can specify a site of the ligand present in a designated region as the main site.

In addition, the main site specifying unit 116 may determine the first threshold value described above through the operation of the user via the operation unit 400, or may determine the first threshold value without depending on the operation of the user (for example, a predetermined value may be used).

[Calculation of Partial Feature Amount]

The partial feature amount calculation unit 120 (processor) calculates a partial feature amount that is a part derived from the main site, in the first feature amount (step S140 in Fig. 4: partial feature amount calculation processing, partial feature amount calculation step). The partial feature amount calculation unit 120 can calculate the partial feature amount, for example, by the following methods.

(1) For a point of a centroid of a main site of the ligand, a three-dimensional AAM descriptor cut out by a sphere centered at that point is used as the partial feature amount.
(2) For a point of a centroid of a main site of the ligand, a three-dimensional AAM descriptor cut out by any region (which may be divided into a plurality of regions) including that point is used as the partial feature amount.
(3) For any point in the vicinity of a main site of the ligand, a three-dimensional AAM descriptor cut out by a sphere centered at that point is used as the partial feature amount.
(4) For any point in the vicinity of a main site of the ligand, a three-dimensional AAM descriptor cut out by any region (which may be divided into a plurality of regions) including that point is used as the partial feature amount.
(5) A difference between a three-dimensional AAM descriptor of the ligand and a three-dimensional AAM descriptor of a virtual compound obtained by removing a main site of the ligand is calculated, a point at which an intensity of the difference is maximum is detected, and a three-dimensional AAM descriptor of the ligand cut out by a sphere centered at that point is used as the partial feature amount.
(6) A difference between a three-dimensional AAM descriptor of the ligand and a three-dimensional AAM descriptor of a virtual compound obtained by removing a main site of the ligand is calculated, a point at which an intensity of the difference is maximum is detected, and a three-dimensional AAM descriptor of the ligand cut out by any region (which may be divided into a plurality of regions) including that point is used as the partial feature amount.

The partial feature amount calculation unit 120 may determine which technique is used to calculate the partial feature amount by the operation of the user via the operation unit 400, or may determine the technique without depending on the operation of the user. In addition, "any point in the vicinity of the main site" or the like may be determined by the operation of the user via the operation unit 400 or may be determined without depending on the operation of the user.

[Calculation of Overall Feature Amount (Second Feature Amount) of Search Candidate Compound]

The feature amount calculation unit 118 (processor) calculates a second feature amount which is a feature amount obtained by quantifying, in a three-dimensional space, a degree of accumulation of one or more amino acids as a probe for the search target compound in a periphery of the search target compound (step S150: second feature amount calculation processing, second feature amount calculation step). This second feature amount is a feature amount derived from the entire search target compound, and hereinafter, may be referred to as an "overall feature amount" of the search target compound.

The feature amount calculation unit 118 can calculate an overall feature amount (second feature amount) of a search candidate compounds by (Method 1) and (Method 2) in the same manner as described above for the overall feature amount (first feature amount) of the ligand. That is, the overall feature amount (second feature amount) of the search candidate compounds is a three-dimensional AAM descriptor of a search compound. It is preferable that the feature amount calculation unit 118 makes the number and combination of amino acids the same between the calculation of the overall feature amount (first feature amount) of the ligand and the calculation of the overall feature amount (second feature amount) of the search candidate compound. In addition, the feature amount calculation unit 118 may calculate the first feature amount and the second feature amount in parallel.

[Calculation of Similarity]

The similarity calculation unit 122 (processor) calculates a similarity between the overall feature amount (second feature amount) for the search target compound and the partial feature amount of the ligand (step S160: similarity calculation processing, similarity calculation step). The similarity calculation unit 122 can calculate the similarity by, for example, the following methods.

(1) A three-dimensional AAM descriptor (overall feature amount (second feature amount) of the search target compound) is calculated for the most stable conformation obtained by executing search calculation (MD, MM, AlphaFold2, or the like) of the most stable conformation of the molecule (here, the search target compound, the same applies hereinafter), a plurality of partial feature amounts created by translating the partial feature amount of the ligand at any distance and rotating the partial feature amount at any angle are calculated, all of cos similarities (cosine similarities, the same applies hereinafter) between the plurality of partial feature amounts and the overall feature amount of the search target compound are calculated, and a maximum value among the cos similarities is calculated as the "similarity".

(2) A three-dimensional AAM descriptor (overall feature amount (second feature amount) of the search target compound) is calculated for the most stable conformation obtained by executing search calculation (MD, MM, AlphaFold2, or the like) of the most stable conformation of the molecule, a plurality of partial feature amounts created by translating the partial feature amount of the ligand at any distance and rotating the partial feature amount at any angle are calculated, all of Euclid distances between the partial feature amounts are calculated, and a minimum value among the Euclid distances (Euclidean distances, the same applies hereinafter) is calculated as the "similarity".

(3) A three-dimensional AAM descriptor (overall feature amount (second feature amount) of the search target compound) is calculated for a plurality of local stable conformations obtained by executing search calculation (MD, MM, AlphaFold2, or the like) of the local stable conformation of the molecule, a plurality of partial feature amounts created by translating the partial feature amount of the ligand at any distance and rotating the partial feature amount at any angle are calculated, all of cos similarities between the partial feature amounts are calculated, and a maximum value among the cos similarities is calculated as the "similarity".

(4) A three-dimensional AAM descriptor (overall feature amount (second feature amount) of the search target compound) is calculated for a plurality of local stable conformations obtained by executing search calculation (MD, MM, AlphaFold2, or the like) of the local stable conformation of the molecule, a plurality of partial feature amounts created by translating the partial feature amount of the ligand at any distance and rotating the partial feature amount at any angle are calculated, all of Euclid distances between the partial feature amounts are calculated, and a minimum value among the Euclid distances is calculated as the "similarity".

The similarity calculation unit 122 may determine which method is used to calculate the similarity by the operation of the user via the operation unit 400, or may determine the method without depending on the operation of the user.

[Output of Relationship between Similarity and Molecular weight/Activity]

The input/output control unit 126 (processor) outputs the similarity for the search target compound and information indicating the molecular weight and/or the activity of the search target compound in association with each other to the recording device (storage unit 200 and the like) and/or the display device (monitor 310 and the like) (step S170: output step, output processing). The processor 110 may acquire information already calculated for the molecular weight and the activity, or may calculate the molecular weight and the activity from the structural information of the search target compound.

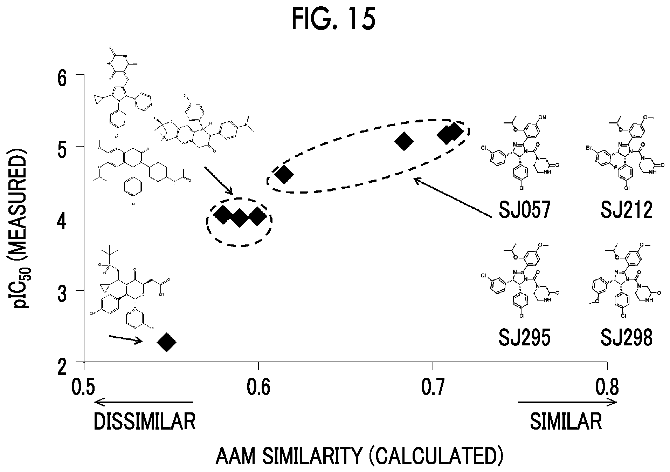

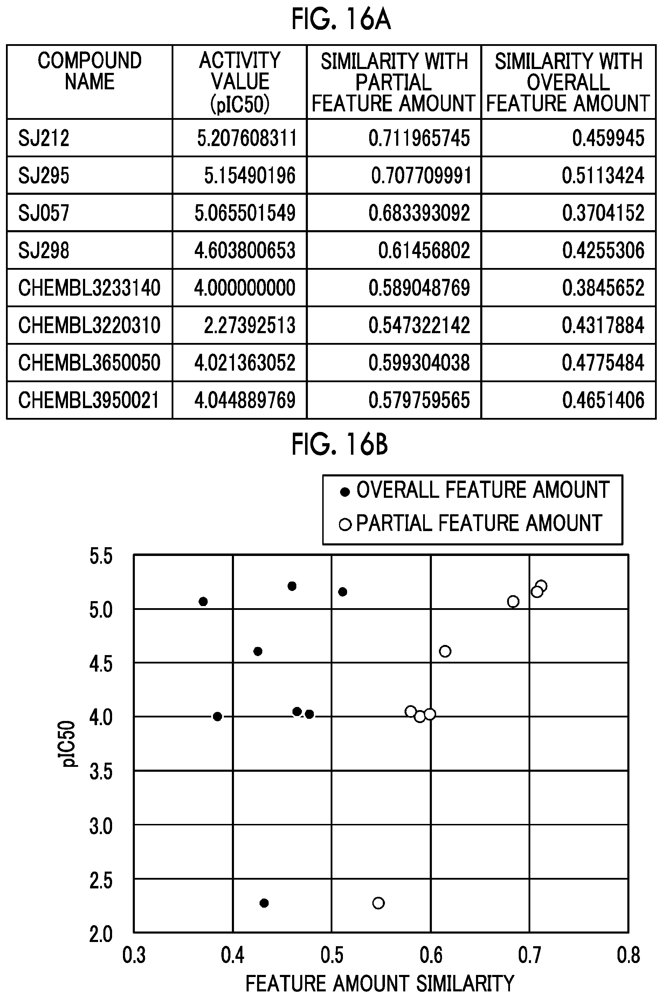

As described above, according to the compound searching device 10 (compound searching device), the compound searching method, and the compound searching program according to the first embodiment, it is possible to provide information for efficiently extracting a compound reduced in molecular weight. As described above, since the reduction in molecular weight is one of the measures for imparting activity and membrane permeability, according to the first aspect, it is possible to efficiently search for a low-molecular-weight compound having activity and membrane permeability. Specific output examples will be described later in Examples (see Figs. 15 and 16, and the like).

[Extraction of Compound]

In addition, the compound extraction unit 124 (processor) may extract a compound satisfying a desired condition with reference to the calculated information (similarity, molecular weight, and activity). For example, the compound extraction unit 124 can extract a compound having a similarity equal to or higher than the second threshold value from the search target compounds (step S180: compound extraction processing, compound extraction step). The compound extraction unit 124 may extract a predetermined number of compounds in descending order of the similarity from the compounds having a similarity equal to or higher than the second threshold value, or may extract all the compounds having a similarity equal to or higher than the second threshold value. The compound extraction unit 124 may determine the number of compounds to be extracted according to the operation of the user.

The compound extraction unit 124 may extract the compound with reference to information (molecular weight, activity) other than the similarity. In addition, the user may extract a desired compound by himself/herself with reference to the information output to the storage unit 200 and/or the monitor 310.

The search target compound having a feature amount (second feature amount) having a high similarity with the partial feature amount (which is a value smaller than the overall feature amount (first feature amount) or has a high possibility of being smaller than the overall feature amount) of the ligand is likely to have a smaller molecular weight than the original ligand. Therefore, according to the compound searching device 10 (compound searching device), the compound searching method, and the compound searching program according to the first embodiment, a compound reduced in molecular weight can be efficiently extracted. As described above, since the reduction in molecular weight is one of the measures for imparting activity and membrane permeability, according to the first embodiment, it is possible to efficiently search for a low-molecular-weight compound having activity and membrane permeability.

[Example]

Specific examples of the compound search will be described in detail.

[Acquisition of Structural Information and Conformational Information, and Specification of Main Site]

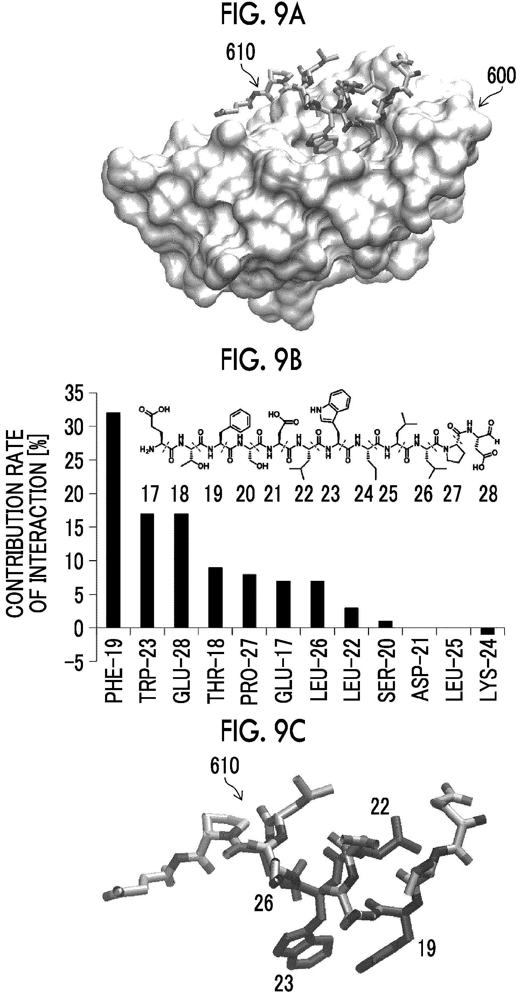

First, the conformation (= bonding conformation) and the main site of the ligand in the formation of a complex with the target protein are specified. In the present example, an X-ray co-crystal structure of a peptide p53-TAD1 (an example of a ligand; hereinafter, may be simply referred to as "p53-TAD1") and a target protein MDMX (an example of a target protein; hereinafter, may be simply referred to as "MDMX") is used (by the structural information acquisition unit 112 and the conformational information acquisition unit 114; structural information acquisition processing/step and conformational information acquisition processing/step). Fig. 9A is a diagram showing a complex of a target protein 600 (MDMX) and a ligand 610 (p53-TAD1).

The bonding energy (bonding force) of p53-TAD1 and MDMX with respect to the X-ray co-crystal structure was calculated by FMO, and a breakdown of the bonding energy for each amino acid residue of p53-TAD1 was obtained (by the main site specifying unit 116; main site specifying processing, main site specifying step). By selecting the amino acid residues such that a ratio of a total value of the breakdown was 60% (first threshold value), amino acid residues 19, 22, 23, and 26 were specified as the main sites (by the main site specifying unit 116; main site specifying processing, main site specifying step). Fig. 9B is a diagram showing a breakdown of bonding energy (bonding force) for each amino acid residue. The main site specifying unit 116 and the input/output control unit 126 can display such a diagram on the monitor 310 (display device) in a case of specifying the main site, and thus the user can easily ascertain the breakdown of the bonding energy for each amino acid residue. In a horizontal axis of Fig. 9B, 19, 23, and the like are amino acid residue numbers, and "PHE", "TRP", and the like indicate original amino acids (PHE: Phenylalanine, TRP: Tryptophan or Tryptophane).

[Identifiable Display of Main Site]

Fig. 9C is a diagram showing a three-dimensional structure of the ligand 610, and shows a state in which the amino acid residues 19, 22, 23, and 26 (one or more amino acid residues) specified as the main sites are displayed in an identifiable manner (specifically, changing a color) on the monitor 310 (display device) by the main site specifying unit 116 and the input/output control unit 126. With such an identifiable display, the user of the compound searching device 10 can easily ascertain the site of the amino acid residue specified as the main site. The identifiable display can be performed not only by a change in color but also by characters, numbers, figures, symbols, or a combination of these with a change in color.

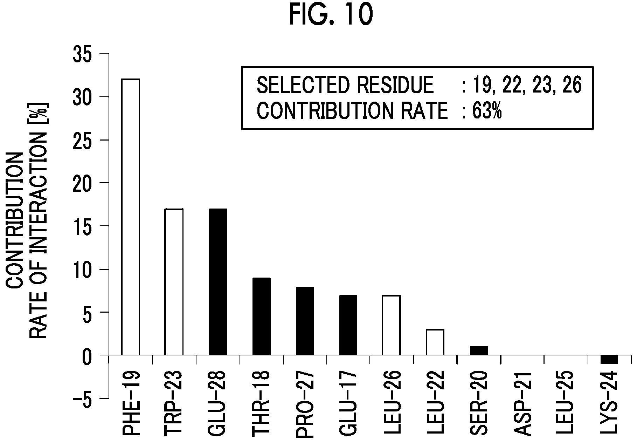

Fig. 10 is a diagram showing a breakdown of bonding energy in amino acid residues specified as the main sites in the diagram shown in Fig. 9B. In Fig. 10, a contribution rate of the bonding energy due to the selected residue is identifiably displayed in a different color from other residues. The identifiable display of the residue can be performed not only by a change in color but also by characters, numbers, figures, symbols, or a combination of these with a change in color, as in a case of the main site. By displaying such a diagram on the monitor 310 by the main site specifying unit 116 and the input/output control unit 126, the user can easily ascertain a relationship between the bonding energy of the residue constituting the main site and the bonding energy of the residue constituting the site other than the main site. Although the contribution of amino acid residues 28, 18, and the like to the bonding energy is also large, in the present example, the main site specifying unit 116 selects amino acid residues 19, 22, 23, and 26 (one or more amino acid residues) in consideration of a distance between the amino acid residues. In a case where discreteness of the selected residues is high (in a case where amino acid residues 28, 18, and the like are also selected in the examples of Figs. 9A, 9B, and 9C and Fig. 10), and the bonding energy (interaction) derived from these residues is to be realized with a single molecule (compound), the molecular weight of such a molecule tends to be large. Therefore, it is preferable to select the amino acid residues in consideration of the distance.

[Calculation of Partial Feature Amount]

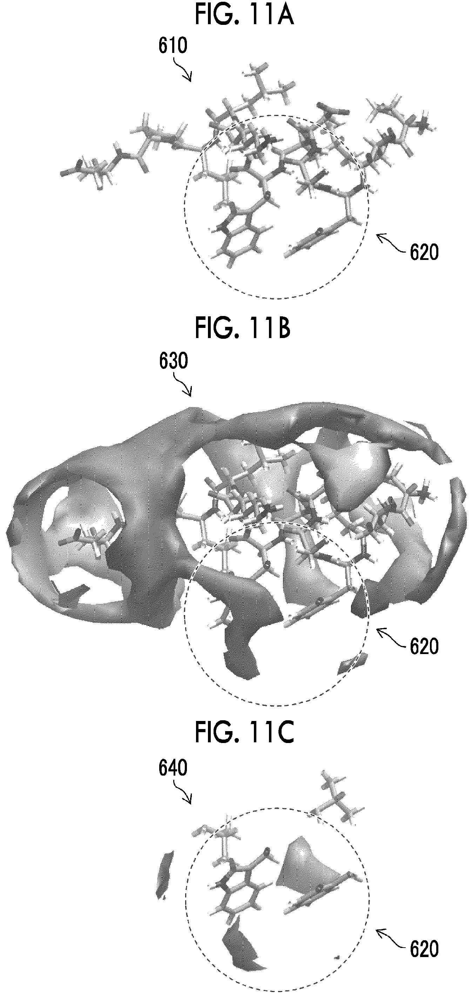

The feature amount calculation unit 118 calculates the three-dimensional AAM descriptor (first feature amount, overall feature amount) for the bonding conformation of p53-TAD1 (ligand) by the above-described technique (first feature amount calculation processing, first feature amount calculation step), and the partial feature amount calculation unit 120 specifies a part (partial feature amount) derived from the main site of p53-TAD1 in the calculated three-dimensional AAM descriptor (partial feature amount calculation step, partial feature amount calculation processing). Figs. 11A, 11B, and 11C are diagrams showing a state in which a partial feature amount is calculated. Fig. 11A shows a state in which a point of a centroid of amino acid residues 19, 22, 23, and 26, which are specified as the main sites in p53-TAD1 (ligand 610), is detected, and a sphere 620 having a radius of 7 Å (7 angstroms) from that point is generated. The main site specifying unit 116 and the partial feature amount calculation unit 120 can calculate the partial feature amount by cutting out a part of the three-dimensional AAM descriptor (overall feature amount, first feature amount) that is included in the sphere 620. That is, the sphere 620 indicates the number, position, size, and shape of regions for calculating the partial feature amount.

The region for cutting out the partial feature amount is not limited to one, and the shape is not limited to a sphere. In addition, the size of the region is not limited to 7 Å. The partial feature amount calculation unit 120 may determine at least one of the number, position, size, or shape of a region for cutting out the partial feature amount according to the operation of the user via the operation unit 400 (input device), or may set them without depending on the operation of the user.

Fig. 11B shows a state in which the feature amount calculation unit 118, the partial feature amount calculation unit 120, and the input/output control unit 126 (processor) display, on the monitor 310 (display device), the three-dimensional structure of p53-TAD1 (ligand), a distribution of three-dimensional AAM descriptors 630 (first feature amounts) in the periphery of the ligand, and the sphere 620 (region for calculating or cutting out the partial feature amount) in a superimposed manner. In addition, Fig. 11C is a diagram showing a calculated partial feature amount 640. The user can easily ascertain a relationship between the overall feature amount of the ligand and the partial feature amount calculation region, and the distribution of the calculated partial feature amounts by these displays.

The feature amount calculation unit 118, the partial feature amount calculation unit 120, and the input/output control unit 126 may display information indicating a ratio of the partial feature amount to the overall feature amount (first feature amount) on the monitor 310 (display device) and/or store the information in the storage unit 200.

The partial feature amount calculation unit 120 can calculate the partial feature amount by $g_a(r)\theta a(r)$, which is a product of the distribution function $g_a(r)$ representing the three-dimensional AAM descriptor and $\theta a(r)$ representing a definition range of the partial feature amount (for example, the sphere 620) (partial feature amount calculation processing, partial feature amount calculation step). $\theta a(r)$ is a function of 1 inside a sphere having a radius of 7 Å, which is a definition range, and 0 outside the sphere. The subscript a means a kind of amino acid, and the argument r = (x, y, z) means a position in a three-dimensional space. In a case where the number of kinds of amino acids is N, the subscript a is a = 1, 2, ..., N. As described above, the number of amino acids as the probe may be 1 or more, but in the present example, the overall feature amount and the partial feature amount are calculated using all the amino acids (20 kinds) as the probe.

[Calculation of Overall Feature Amount of Search Target Compound]

Next, a three-dimensional AAM descriptor (overall feature amount, second feature amount) is calculated for the conformation of the molecule to be searched for or designed, and the similarity with the partial feature amount of the ligand is obtained. Figs. 12A, 12B, and 12C are diagrams showing a state in which the overall feature amount of the search target compound is calculated. In the present example, a three-dimensional structure as shown in Fig. 12B was acquired from a structural formula of a search target compound (hereinafter, may be referred to as a "compound A") illustrated in Fig. 12A, the most stable conformation thereof was calculated by MO calculation, and a three-dimensional AAM descriptor was calculated for this conformation (Fig. 12C). These pieces of processing can be executed by the structural information acquisition unit 112, the feature amount calculation unit 118, and the like as described above.

[Calculation of Similarity]

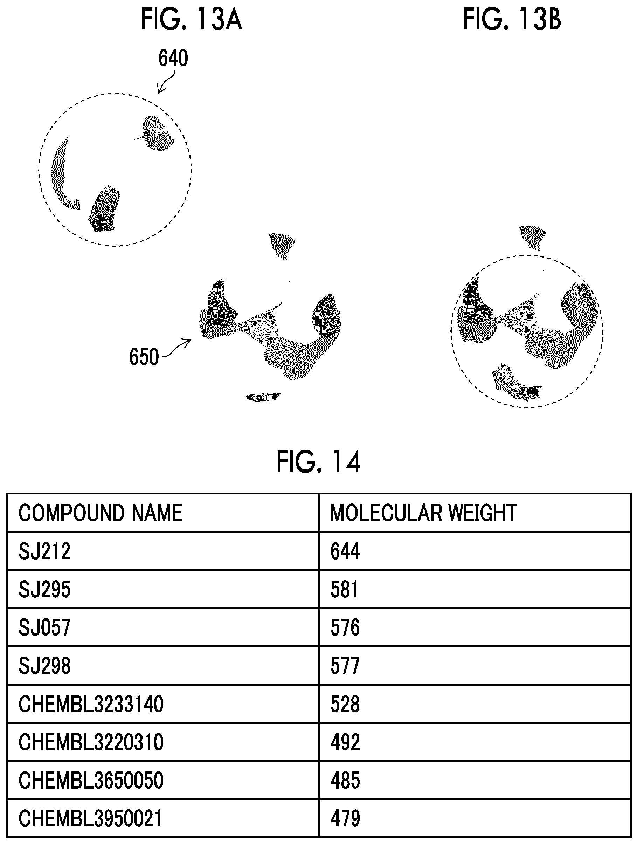

The partial feature amount calculation unit 120 created a plurality of partial feature amounts of p53-TAD1 by translating and rotating the partial feature amount of p53-TAD1. Here, the partial feature amount calculation unit 120 performed the translation of the partial feature amount of p53-TAD1 in increments of 1 Å in each of orthogonal coordinates X, Y, and Z in the three-dimensional space, and performed the rotation in increments of 10° in each of Euler angles $\alpha$, $\beta$, and $\gamma$. The partial feature amount calculation unit 120 calculated the cos similarities between an overall feature amount of the compound A and a plurality of partial feature amounts of p53-TAD1, and adopted the maximum cos similarity among the cos similarities as the final "similarity". The calculation of the cos similarity was performed in a sphere having a radius of 7 Å, which is the definition range of the partial feature amount. Fig. 13A and 13B are diagrams showing a state in which the similarity is calculated. Fig. 13A shows a state in which a cos similarity between the partial feature amount 640 of p53-TAD1 and an overall feature amount 650 of the compound A is zero, and Fig. 13B shows a state in which the cos similarity is maximum (0.71).

The overall feature amount of the compound A and the partial feature amount created by the translation and rotation are represented by $G_a(r)$ and $g_a(Rr + s)\theta_a(Rr + s)$, respectively, on a computer. Here, R is a rotation matrix and is represented by Euler angles $\alpha$, $\beta$, and $\gamma$, and s is a translation vector and is represented by X, Y, and Z (R = R($\alpha$, $\beta$, $\gamma$), s = (X, Y, Z)). In addition, the cos similarity between the overall feature amount of the compound A and the partial feature amount created by the translation and rotation can be represented by the following Equations (2) to (6) as a function of R and s.

$$\text{cos similarity } (R,s) \equiv$$

$$\frac{1}{N}\sum_{a=1}^{N} \frac{\iiint d^3r\left[G_a(r)-G_a^{(1i)}(R,s)\right]\left[g_a(Rr+s)-g_a^{(1i)}(R,s)\right]\theta_a(Rr+s)}{\sqrt{G_a^{(2i)}(R,s)-G_a^{(1i)}(R,s)G_a^{(1i)}(R,s)}\sqrt{g_a^{(2i)}(R,s)-g_a^{(1i)}(R,s)g_a^{(1i)}(R,s)}\iiint d^3r\theta_a(Rr+s)}$$

$$\cdots (2)$$

$$G_a^{(1)}(R,s) \equiv \frac{\iiint d^3r\, G_a(r)\theta_a(Rr+s)}{\iiint d^3r\, \theta_a(Rr+s)} \qquad \cdots (3)$$

$$G_a^{(2)}(R,s) \equiv \frac{\iiint d^3r\, G_a(r)G_a(r)\theta_a(Rr+s)}{\iiint d^3r\, \theta_a(Rr+s)} \qquad \cdots (4)$$

$$g_a^{(1)}(R,s) \equiv \frac{\iiint d^3r\, g_a(Rr+s)\theta_a(Rr+s)}{\iiint d^3r\, \theta_a(Rr+s)} \qquad \cdots (5)$$

$$g_a^{(2)}(R,s) \equiv \frac{\iiint d^3r\, g_a(Rr+s)g_a(Rr+s)\theta_a(Rr+s)}{\iiint d^3r\, \theta_a(Rr+s)} \qquad \cdots (6)$$

In addition, the final similarity (AAM similarity) is calculated by the following Equation (7).

$$\text{AAM similarity} = \max_{(R,s)}\{\cos \text{ similarity } (R,s)\} \qquad \cdots (7)$$

In the present example, the cos similarity is calculated, but the similarity based on the Euclid distance may be calculated as described above. In addition, in the present example, the similarity based on the most stable conformation of the compound A is calculated, but the similarity based on the local stable conformation may be calculated.

[Result]

The AAM similarity (Equation (7)) between three-dimensional AAM descriptors (overall feature amounts, second feature amounts) of eight compounds (search target compounds corresponding to the "compound A" described above; names and molecular weights are shown in a table of Fig. 14) and the partial feature amount of p53-TAD1 was calculated and compared with inhibitory activity $IC_{50}$ against MDMX in experiments. This result is shown in Fig. 15. In the diagram, $pIC_{50} = -\log IC_{50}$. As shown in the diagram, the higher the AAM similarity of the compound, the higher the inhibitory activity ($pIC_{50}$). Since the high-activity compound had a molecular weight of about 500 to 600, the molecular weight could be reduced to about 900 to 1,000 as compared with the molecular weight of about 1,500 of p53-TAD1.

Based on this result, the compound extraction unit 124 can extract a compound having a similarity equal to or higher than the second threshold value (for example, equal to or higher than 0.6, equal to or higher than 0.65, and the like) (compound extraction step, compound extraction processing), and the input/output control unit 126 can output information indicating the extracted compound to the storage unit 200 (recording device) and/or the monitor 310 (display device). The compound extraction unit 124 may set the "second threshold value" based on the operation of the user or may set the "second threshold value" without depending on the operation of the user.

In addition, the compound extraction unit 124 may extract the compound with reference to information (molecular weight and activity) other than the similarity. Furthermore, the user may extract a desired compound by himself/herself with reference to the information output to the storage unit 200 and/or the monitor 310.

[Comparative Example]

In the above-described example, although the similarity between the "partial feature amount" of the ligand and the overall feature amount (second feature amount) of the search target compound is calculated, a comparison result in a case where a similarity between the "overall feature amount (first feature amount)" of the ligand and the overall feature amount of the search target compound is considered is shown in Figs. 16A and 16B. Fig. 16A is a table showing, regarding the target compounds (the same as in the above-described example), a name, an activity value, a similarity with the partial feature amount of the ligand, and a similarity with the overall feature amount of the ligand, and Fig. 16B is a graph showing a relationship between the similarity of the feature amount and the inhibitory activity ($pIC_{50}$).

As shown in Figs. 16A and 16B, compounds (SJ212 to SJ298) having a high similarity with the partial feature amount of the ligand also have a high activity value, but compounds (particularly CHEMBL3650050 and CHEMBL3950021) having a high similarity with the overall feature amount do not necessarily have a high activity value. Therefore, from the results of Comparative Example, it is found that it is effective to pay attention to the similarity between the overall feature amount of the search target compound and the partial feature amount of the ligand in order to extract a compound having a high activity value.

[Regarding Targets to which Present Invention is Applicable]

According to the compound searching device, the compound searching method, and the compound searching program of the embodiments of the present invention described above, it is possible to efficiently search for a low-molecular-weight compound having activity and membrane permeability. In addition, such a low-molecular-weight compound can be used as a candidate compound for a new drug.

In addition, the compound searching device, the compound searching method, and the compound searching program of the embodiments of the present invention can also be applied to a composition of a culture medium of a cell or the like. In the field of the culture medium of cells, fibroblast growth factor(s) (FGF) of a protein and the like are used as culture medium additives, and FGF promotes cell growth by bonding to a fibroblast growth factor receptor (FGFR), which is a receptor of the protein. However, since FGF is expensive, there is a demand for cost reduction. Therefore, it has been considered to use a peptide or a low-molecular-weight compound that is cheaper and bonds to FGFR to reproduce the cell growth promoting effect instead of the expensive FGF. However, no peptide or low-molecular-weight compound that exhibits a sufficient effect has been found. By applying the technique for reduction in molecular weight of the present invention to FGF, there is a possibility that a peptide or a low-molecular-weight compound that binds to FGFR and reproduces the cell growth promoting effect can be found.

Hereinbefore, the embodiment of the present invention has been described, but the present invention is not limited to the above-described aspects, and various modifications can be made.

[Appendices]

The present invention includes the following aspects including the aspects described in the claims.

(Appendix 1)

A compound searching device according to Appendix 1 is a compound searching device comprising a processor, in which the processor is configured to: acquire information indicating a three-dimensional structure of a search target compound; acquire information indicating a conformation of a ligand in a complex structure of the ligand and a target protein; calculate a first feature amount that is a feature amount obtained by quantifying, in a three-dimensional space, a degree of accumulation of one or more amino acids as a probe for the ligand in a periphery of the ligand with respect to the conformation of the ligand; specify, as a main site, a site of a part of the ligand, of which a proportion in a bonding force of the ligand to the target protein is equal to or higher than a first threshold value; calculate a partial feature amount that is a part derived from the main site, in the first feature amount; calculate a second feature amount that is a feature amount obtained by quantifying, in a three-dimensional space, a degree of accumulation of one or more amino acids as a probe for the search target compound in a periphery of the search target compound; calculate a similarity between the second feature amount for the search target compound and the partial feature amount of the ligand; and output the similarity of the search target compound and information indicating a molecular weight and/or activity of the search target compound in association with each other to a recording device and/or a display device.

(Appendix 2)

A compound searching device according to Appendix 2 is the compound searching device according to Appendix 1, in which the processor is configured to, in specifying the main site, acquire any one of a result of bonding energy calculation using a complex structure of the ligand and the target protein, information indicating a structural activity correlation between the ligand and a ligand analogous substance, or the first feature amount for a local stable conformation of the ligand from a non-transitory and tangible recording medium, as the information indicating the bonding force, and specify the main site based on the acquired information.

(Appendix 3)

A compound searching device according to Appendix 3 is the compound searching device according to Appendix 1 or 2, in which the processor is configured to, in specifying the main site, select one or more amino acid residues in which a ratio of a total value of a breakdown of a bonding force due to each amino acid residue of the ligand to the bonding force of the ligand is equal to or higher than a designated value, and specify a site of the one or more amino acid residues in the ligand as the main site.

(Appendix 4)

A compound searching device according to Appendix 4 is the compound searching device according to Appendix 3, in which the processor is configured to, in specifying the main site, select the one or more amino acid residues in consideration of a distance between the amino acid residues.

(Appendix 5)

A compound searching device according to Appendix 5 is the compound searching device according to any one of Appendices 1 to 4, in which the processor is configured to, in specifying the main site, specify a site of the ligand present in a designated region as the main site.

(Appendix 6)

A compound searching device according to Appendix 6 is the compound searching device according to Appendix 5, in which the processor is configured to, in specifying the main site, designate the region based on a distribution of the first feature amount in a periphery of the ligand.

(Appendix 7)

A compound searching device according to Appendix 7 is the compound searching device according to Appendix 5, in which the processor is configured to, in specifying the main site, determine at least one of the number, position, size, or shape of the region according to an operation of a user via an input device.

(Appendix 8)

A compound searching device according to Appendix 8 is the compound searching device according to any one of Appendices 5 to 7, in which the processor is configured to, in specifying the main site, display, on the display device, information indicating a three-dimensional structure of the ligand, a distribution of the first feature amount in a periphery of the ligand, and the number, position, size, and shape of the region in a superimposed manner.

(Appendix 9)

A compound searching device according to Appendix 9 is the compound searching device according to any one of Appendices 1 to 8, in which the processor is configured to display information indicating a ratio of the partial feature amount to the first feature amount on the display device.

(Appendix 10)

A compound searching device according to Appendix 10 is the compound searching device according to any one of Appendices 3 to 9, in which the processor is configured to, in specifying the main site, display, on the display device, the site of the one or more amino acid residues specified as the main site in a three-dimensional structure of the ligand in an identifiable manner.

(Appendix 11)

A compound searching device according to Appendix 11 is the compound searching device according to any one of Appendices 1 to 10, in which the processor is configured to, in specifying the main site, calculate a breakdown of bonding energy between the ligand and the target protein for each amino acid residue constituting the ligand, and display the calculated breakdown on the display device for each amino acid residue.

(Appendix 12)

A compound searching device according to Appendix 12 is the compound searching device according to any one of Appendices 1 to 11, in which the processor is configured to: calculate a plurality of partial feature amounts by translating and/or rotating the partial feature amount of the ligand in the calculation of the partial feature amount; and calculate the similarity based on a cosine similarity or a Euclidean distance between the plurality of partial feature amounts and the second feature amount for the search target compound in the calculation of the similarity.

(Appendix 13)

A compound searching device according to Appendix 13 is the compound searching device according to any one of Appendices 1 to 12, in which the processor is configured to, in the outputting, extract a compound of which the similarity is equal to or higher than a second threshold value from the search target compounds, and output information indicating the extracted compound to the recording device and/or the display device.

(Appendix 14)

A compound searching method according to Appendix 14 is a compound searching method executed by a compound searching device including a processor, the method comprising: via the processor, acquiring information indicating a three-dimensional structure of a search target compound; acquiring information indicating a conformation of a ligand in a complex structure of the ligand and a target protein; calculating a first feature amount that is a feature amount obtained by quantifying, in a three-dimensional space, a degree of accumulation of one or more amino acids as a probe for the ligand in a periphery of the ligand with respect to the conformation of the ligand; specifying, as a main site, a site of a part of the ligand, of which a proportion in a bonding force of the ligand to the target protein is equal to or higher than a first threshold value; calculating a partial feature amount that is a part derived from the main site, in the first feature amount; calculating a second feature amount that is a feature amount obtained by quantifying, in a three-dimensional space, a degree of accumulation of one or more amino acids as a probe for the search target compound in a periphery of the search target compound; calculating a similarity between the second feature amount for the search target compound and the partial feature amount of the ligand; and outputting the similarity of the search target compound and information indicating a molecular weight and/or activity of the search target compound in association with each other to a recording device and/or a display device.
The compound searching method according to Appendix 14 may have the same configurations as those of the aspects of Appendices 2 to 13.

(Appendix 15)

A compound searching program according to Appendix 15 is a compound searching program for causing a compound searching device including a processor to execute a compound searching method, the compound searching method comprising: via the processor, acquiring information indicating a three-dimensional structure of a search target compound; acquiring information indicating a conformation of a ligand in a complex structure of the ligand and a target protein; calculating a first feature amount that is a feature amount obtained by quantifying, in a three-dimensional space, a degree of accumulation of one or more amino acids as a probe for the ligand in a periphery of the ligand with respect to the conformation of the ligand; specifying, as a main site, a site of a part of the ligand, of which a proportion in a bonding force of the ligand to the target protein is equal to or higher than a first threshold value; calculating a partial feature amount that is a part derived from the main site, in the first feature amount; calculating a second feature amount that is a feature amount obtained by quantifying, in a three-dimensional space, a degree of accumulation of one or more amino acids as a probe for the search target compound in a periphery of the search target compound; calculating a similarity between the second feature amount for the search target compound and the partial feature amount of the ligand; and outputting the similarity of the search target compound and information indicating a molecular weight and/or activity of the search target compound in association with each other to a recording device and/or a display device.
The compound searching program according to Appendix 15 may have the same configurations as those of the aspects of Appendices 2 to 13.
In addition, a non-transitory and tangible recording medium (for example, various magneto-optical recording devices and semiconductor memories) on which a computer-readable code of the program of these aspects is recorded can also be mentioned as an aspect of the present invention. The "non-transitory and tangible recording medium" described above does not include a non-tangible recording medium such as the carrier wave signal itself and the propagation signal itself.

Explanation of References

10: compound searching device
100: processing unit
110: processor
112: structural information acquisition unit

114: conformational information acquisition unit
116: main site specifying unit
118: feature amount calculation unit
120: partial feature amount calculation unit
122: similarity calculation unit
124: compound extraction unit
126: input/output control unit
128: communication control unit
130: ROM
140: RAM
200: storage unit
202: structural information
204: conformational information
206: feature amount
208: search condition
210: search result
300: display unit
310: monitor
400: operation unit
410: keyboard
420: mouse
500: external server
510: external database
600: target protein
610: ligand
620: sphere
630: three-dimensional AAM descriptor
640: partial feature amount
650: overall feature amount
MDMX: target protein
NW: network
S100 to S240: each step of compound searching method

**Claims**

1. A compound searching device comprising a processor,
   wherein the processor is configured to:

   acquire information indicating a three-dimensional structure of a search target compound;
   acquire information indicating a conformation of a ligand in a complex structure of the ligand and a target protein;
   calculate a first feature amount that is a feature amount obtained by quantifying, in a three-dimensional space, a degree of accumulation of one or more amino acids as a probe for the ligand in a periphery of the ligand with respect to the conformation of the ligand;
   specify, as a main site, a site of a part of the ligand, of which a proportion in a bonding force of the ligand to the target protein is equal to or higher than a first threshold value;
   calculate a partial feature amount that is a part derived from the main site, in the first feature amount;
   calculate a second feature amount that is a feature amount obtained by quantifying, in a three-dimensional space, a degree of accumulation of one or more amino acids as a probe for the search target compound in a periphery of the search target compound;
   calculate a similarity between the second feature amount for the search target compound and the partial feature amount of the ligand; and
   output the similarity for the search target compound and information indicating a molecular weight and/or activity of the search target compound in association with each other to a recording device and/or a display device.

2. The compound searching device according to claim 1,

   wherein the processor is configured to, in specifying the main site,

acquire any one of a result of bonding energy calculation using a complex structure of the ligand and the target protein, information indicating a structural activity correlation between the ligand and a ligand analogous substance, or the first feature amount for a local stable conformation of the ligand from a non-transitory and tangible recording medium, as the information indicating the bonding force, and
specify the main site based on the acquired information.

3. The compound searching device according to claim 1 or 2,

wherein the processor is configured to, in specifying the main site,
select one or more amino acid residues in which a ratio of a total value of a breakdown of a bonding force due to each amino acid residue of the ligand to the bonding force of the ligand is equal to or higher than the first threshold value, and specify a site of the one or more amino acid residues in the ligand as the main site.

4. The compound searching device according to claim 3,

wherein the processor is configured to, in specifying the main site,
select the one or more amino acid residues in consideration of a distance between the amino acid residues.

5. The compound searching device according to claim 1 or 2,

wherein the processor is configured to, in specifying the main site,
specify a site of the ligand present in a designated region as the main site.

6. The compound searching device according to claim 5, wherein the processor is configured to, in specifying the main site,
designate the region based on a distribution of the first feature amount in a periphery of the ligand.

7. The compound searching device according to claim 5,

wherein the processor is configured to, in specifying the main site,
determine at least one of the number, position, size, or shape of the region according to an operation of a user via an input device.

8. The compound searching device according to claim 5,

wherein the processor is configured to, in specifying the main site,
display, on the display device, information indicating a three-dimensional structure of the ligand, a distribution of the first feature amount in a periphery of the ligand, and the number, position, size, and shape of the region in a superimposed manner.

9. The compound searching device according to claim 1 or 2,
wherein the processor is configured to display information indicating a ratio of the partial feature amount to the first feature amount on the display device.

10. The compound searching device according to claim 3,

wherein the processor is configured to, in specifying the main site,
display, on the display device, the site of the one or more amino acid residues specified as the main site in a three-dimensional structure of the ligand in an identifiable manner.

11. The compound searching device according to claim 1 or 2,

wherein the processor is configured to, in specifying the main site,
calculate a breakdown of bonding energy between the ligand and the target protein for each amino acid residue constituting the ligand, and display the calculated breakdown on the display device for each amino acid residue.

12. The compound searching device according to claim 1 or 2,
wherein the processor is configured to:

calculate a plurality of partial feature amounts by translating and/or rotating the partial feature amount of the ligand in the calculation of the partial feature amount; and

calculate the similarity based on a cosine similarity or a Euclidean distance between the plurality of partial feature amounts and the second feature amount for the search target compound in the calculation of the similarity.

13. The compound searching device according to claim 1 or 2,

wherein the processor is configured to, in the outputting,

extract a compound of which the similarity is equal to or higher than a second threshold value from the search target compounds, and output information indicating the extracted compound to the recording device and/or the display device.

14. A compound searching method executed by a compound searching device including a processor, the method comprising:

via the processor,

acquiring information indicating a three-dimensional structure of a search target compound;

acquiring information indicating a conformation of a ligand in a complex structure of the ligand and a target protein;

calculating a first feature amount that is a feature amount obtained by quantifying, in a three-dimensional space, a degree of accumulation of one or more amino acids as a probe for the ligand in a periphery of the ligand with respect to the conformation of the ligand;

specifying, as a main site, a site of a part of the ligand, of which a proportion in a bonding force of the ligand to the target protein is equal to or higher than a first threshold value;

calculating a partial feature amount that is a part derived from the main site, in the first feature amount;

calculating a second feature amount that is a feature amount obtained by quantifying, in a three-dimensional space, a degree of accumulation of one or more amino acids as a probe for the search target compound in a periphery of the search target compound;

calculating a similarity between the second feature amount for the search target compound and the partial feature amount of the ligand; and

outputting the similarity for the search target compound and information indicating a molecular weight and/or activity of the search target compound in association with each other to a recording device and/or a display device.

15. A compound searching program for causing a compound searching device including a processor to execute a compound searching method, the compound searching method comprising:

via the processor,

acquiring information indicating a three-dimensional structure of a search target compound;

acquiring information indicating a conformation of a ligand in a complex structure of the ligand and a target protein;

calculating a first feature amount that is a feature amount obtained by quantifying, in a three-dimensional space, a degree of accumulation of one or more amino acids as a probe for the ligand in a periphery of the ligand with respect to the conformation of the ligand;

specifying, as a main site, a site of a part of the ligand, of which a proportion in a bonding force of the ligand to the target protein is equal to or higher than a first threshold value;

calculating a partial feature amount that is a part derived from the main site, in the first feature amount;

calculating a second feature amount that is a feature amount obtained by quantifying, in a three-dimensional space, a degree of accumulation of one or more amino acids as a probe for the search target compound in a periphery of the search target compound;

calculating a similarity between the second feature amount for the search target compound and the partial feature amount of the ligand; and

outputting the similarity for the search target compound and information indicating a molecular weight and/or activity of the search target compound in association with each other to a recording device and/or a display device.

16. A non-transitory computer-readable recording medium on which the program according to claim 15 is recorded.

# FIG. 1

# FIG. 2

100

110

STRUCTURAL INFORMATION
ACQUISITION UNIT — 112

CONFORMATIONAL INFORMATION
ACQUISITION UNIT — 114

MAIN SITE SPECIFYING UNIT — 116

FEATURE AMOUNT
CALCULATION UNIT — 118

PARTIAL FEATURE AMOUNT
CALCULATION UNIT — 120

SIMILARITY CALCULATION UNIT — 122

COMPOUND EXTRACTION UNIT — 124

INPUT/OUTPUT CONTROL UNIT — 126

COMMUNICATION CONTROL UNIT — 128

ROM — 130

RAM — 140

# FIG. 3

200

STRUCTURAL INFORMATION ~202

CONFORMATIONAL INFORMATION ~204

FEATURE AMOUNT ~206

SEARCH CONDITION ~208

SEARCH RESULT ~210

## FIG. 4

START

ACQUIRE STRUCTURAL INFORMATION OF SEARCH TARGET COMPOUND ~S100

ACQUIRE CONFORMATIONAL INFORMATION OF LIGAND ~S110

CALCULATE OVERALL FEATURE AMOUNT OF LIGAND ~S120

SPECIFY SITE OF PART OF LIGAND AS MAIN SITE ~S130

CALCULATE PARTIAL FEATURE AMOUNT DERIVED FROM MAIN SITE ~S140

CALCULATE OVERALL FEATURE AMOUNT OF SEARCH TARGET COMPOUND ~S150

CALCULATE SIMILARITY BETWEEN PARTIAL FEATURE AMOUNT OF LIGAND AND OVERALL FEATURE AMOUNT OF SEARCH TARGET COMPOUND ~S160

OUTPUT SIMILARITY AND INFORMATION INDICATING MOLECULAR WEIGHT AND/OR ACTIVITY IN ASSOCIATION WITH EACH OTHER ~S170

EXTRACT COMPOUND HAVING SIMILARITY EQUAL TO OR HIGHER THAN THRESHOLD VALUE FROM SEARCH TARGET COMPOUNDS ~S180

END

# FIG. 5

START

INPUT STRUCTURAL FORMULA OF COMPOUND — S200

THREE-DIMENSIONALIZE INPUT STRUCTURAL FORMULA — S210

CALCULATE SPATIAL DISTRIBUTION $\Delta G_{a\mu}(r)$ OF FREE ENERGY FELT BY EACH ATOM " $\mu$ " OF AMINO ACID "a" $(r = (x, y, z))$ — S220

CALCULATE DISTRIBUTION FUNCTION $g_{a\mu}(r)$ OF EACH ATOM " $\mu$ " OF AMINO ACID "a" FROM $\Delta G_{a\mu}(r)$ — S230

CALCULATE DISTRIBUTION FUNCTION $g_a(r)$ OF CENTROID OF AMINO ACID FROM DISTRIBUTION FUNCTION $g_{a\mu}(r)$ — S240

END

FIG. 6A

FIG. 6B

FIG. 7A

FIG. 7B

# FIG. 8

| | THREE-DIMENSIONAL AAM DESCRIPTOR (LEVEL SURFACE WITH RESPECT TO THRESHOLD VALUE) | THREE-DIMENSIONAL AAM DESCRIPTOR (LEVEL SURFACE WITH RESPECT TO THRESHOLD VALUE) AND THREE-DIMENSIONAL STRUCTURE OF COMPOUND |
|---|---|---|
| DIRECTION 1 | | |
| DIRECTION 2 | | |
| DIRECTION 3 | | |

## FIG. 9A

## FIG. 9B

## FIG. 9C

FIG. 10

SELECTED RESIDUE : 19, 22, 23, 26
CONTRIBUTION RATE : 63%

## FIG. 11A

610

620

## FIG. 11B

630

620

## FIG. 11C

640

620

FIG. 12A

FIG. 12B

FIG. 12C

## FIG. 13A

640

650

## FIG. 13B

## FIG. 14

| COMPOUND NAME | MOLECULAR WEIGHT |
|---|---|
| SJ212 | 644 |
| SJ295 | 581 |
| SJ057 | 576 |
| SJ298 | 577 |
| CHEMBL3233140 | 528 |
| CHEMBL3220310 | 492 |
| CHEMBL3650050 | 485 |
| CHEMBL3950021 | 479 |

# FIG. 15

## FIG. 16A

| COMPOUND NAME | ACTIVITY VALUE (pIC50) | SIMILARITY WITH PARTIAL FEATURE AMOUNT | SIMILARITY WITH OVERALL FEATURE AMOUNT |
|---|---|---|---|
| SJ212 | 5.207608311 | 0.711965745 | 0.459945 |
| SJ295 | 5.15490196 | 0.707709991 | 0.5113424 |
| SJ057 | 5.065501549 | 0.683393092 | 0.3704152 |
| SJ298 | 4.603800653 | 0.61456802 | 0.4255306 |
| CHEMBL3233140 | 4.000000000 | 0.589048769 | 0.3845652 |
| CHEMBL3220310 | 2.27392513 | 0.547322142 | 0.4317884 |
| CHEMBL3650050 | 4.021363052 | 0.599304038 | 0.4775484 |
| CHEMBL3950021 | 4.044889769 | 0.579759565 | 0.4651406 |

## FIG. 16B

● OVERALL FEATURE AMOUNT
○ PARTIAL FEATURE AMOUNT

pIC50 vs FEATURE AMOUNT SIMILARITY

## INTERNATIONAL SEARCH REPORT

| International application No. |
|---|
| **PCT/JP2023/025264** |

**A. CLASSIFICATION OF SUBJECT MATTER**

*G16B 15/30*(2019.01)i
FI: G16B15/30

According to International Patent Classification (IPC) or to both national classification and IPC

**B. FIELDS SEARCHED**

Minimum documentation searched (classification system followed by classification symbols)

G16B15/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2020/203551 A1 (FUJIFILM CORPORATION) 08 October 2020 (2020-10-08) entire text, all drawings | 1-16 |
| A | JP 2017-174057 A (UNIV OSAKA) 28 September 2017 (2017-09-28) entire text, all drawings | 1-16 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 July 2023** | **08 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

| | International application No. |
|---|---|
| | **PCT/JP2023/025264** |

| Patent document<br>cited in search report | Publication date<br>(day/month/year) | Patent family member(s) | Publication date<br>(day/month/year) |
|---|---|---|---|
| WO 2020/203551 A1 | 08 October 2020 | US 2022/0068441 A1<br>entire text, all drawings<br>EP 3951785 A1<br>entire text, all drawings<br>CN 113678205 A1<br>entire text, all drawings | |
| JP 2017-174057 A | 28 September 2017 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009148192 A
- WO 2010044485 A
- WO 2010128685 A
- WO 2006099178 A
- JP 6826672 B

**Non-patent literature cited in the description**

- **DOUGLAS R. CARY et al.** Constrained Peptides in Drug Discovery and Development. *Journal of Synthetic Organic Chemistry*, 29 July 2022, vol. 75
- **GERD HUMMEL et al.** Translating peptides into small molecules. *Molecular BioSystems*, 29 July 2022, vol. 2, 499-508